# EUROPEAN PATENT APPLICATION

(11) **EP 1 591 107 A1**
(43) Date of publication of application: **02.11.2005**
(21) Application number: 04010075.2
(22) Date of filing: 28.04.2004
(51) Int. Cl.: A61K 9/20, A61K 47/36, A61K 47/14

(54) **Process for the selective increase of a release rate of an active material from a pharmaceutical composition**

(71) Applicant: Pfizer GmbH Arzneimittelwerk Gödecke, 79090 Freiburg (DE)
(72) Inventor: Knapp, Armin, Dr., 79288 Gottenheim (DE); Ganzmann, Tanja, 79211 Denzlingen (DE)
(74) Representative: Tesch, Rudolf

(57) **Abstract**

The present invention provides a process for a selective increase of a release rate in a later stage of the release period of an active material from a controlled release pharmaceutical composition comprising an active material and a lipid material embedding said active material, comprising the step of:
adding a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, in an amount of 3 to 20 percent by weight, based on the entire weight of the controlled release pharmaceutical composition, to a lipophilic matrix comprising an active material embedded in a lipid material; and a controlled release pharmaceutical composition having such a modified release profile comprising an active material, a lipid material embedding said active material, and a swellable organic disintegrant mentioned above, in an amount of 3 to 20 percent by weight.

## Description

The present invention relates to a process for a selective increase of a release rate in a later stage of the release period of an active material from a controlled release pharmaceutical composition, and a controlled release pharmaceutical composition showing a selective increase of a release rate of an active material in a later stage of the release period of the active material.

### Background of the Invention

Controlled release pharmaceutical compositions containing active materials are designed to contain higher concentrations of the active material and are prepared in such a manner as to effect controlled release into the gastro-intestinal digestive tract of humans or animals over an extended period of time. Easily absorbed controlled release therapeutic drug dosage forms have inherent advantages over conventional, immediate release dosage forms. The advantages include less frequent dosing of a medicament and resultant patient regime compliance, a more controlled drug blood level response, the possibility of effecting therapeutic action with less ingested drug and the mitigation of side effects. By providing a controlled release of the active material over a period of time, absorbed drug concentration spikes are mitigated or eliminated by effecting a smoother and more controlled blood level response.

For this purpose a controlled release pharmaceutical composition has to meet certain criteria, namely that of causing a delayed dissolution which is effective for an extended period of time. It is also important that such a formulation is simple to make and that the manufacturing process is reproducible and is used for a number of different active materials.

To prepare controlled release pharmaceutical formulations in the form of a solid oral dosage, such as tablets, insoluble plastics (e.g. methyl acrylate-methacrylate, polyvinyl chloride, or polyethylene), a hydrophilic polymer (e.g. methylcellulose, hydroxypropylmethylcellulose, or sodium carboxyl methylcellulose), or fatty compounds (e.g. various waxes, such as camauba wax, or glyceryl stearate) have been utilized.

For example, EP-A-0 068 446 discloses the addition of hydrophilic swellable soluble polymers such as methylcellulose or carboxymethylcellulose, having different viscosity (0.02 - 40 Pa•s) for a time-controlled release of an active material from a melt granulate. In detail, it is disclosed that if hydrophilic methylcellulose and/or carboxymethylcellulose polymers are added in an amount of 0.1 to 10 weight percent, the release rate, which rises as the result of an increase in the viscosity steps of the hydrophilic polymers, can be empirically determined and the desired release characteristic can be adjusted using the values determined.

Flanders et al. (Flanders, P.; Dyer, G.A.; Jordan, D.: The Control of Drug Release from Conventional Melt Granulation Matrices. Drug Development and Industrial Pharmacy, 13(6), 1001-1022 (1987)) describe the use of colloidal silica as a wicking agent for controlling a release of an active material from melt granulate tablets. Hereby, colloidal silica (Aerosil®) has a similar effect as that of the hydrophilic swellable soluble polymers described in EP-A-0 068 446 in that it thickens water to a predetermined consistency.
Thus, colloidal silica equally influences the release rate of an active material from a controlled release composition as from the beginning of the release and thus results in a uniform rise of the release rate curve during the whole period of time of the release of the active material.

Often, however, a uniform rise of the release rate during the entire period of time of the release of an active material is not desired because it might be advantageous if the release rate is specifically manipulated so as to selectively increase the release rate of an active material from a controlled release pharmaceutical composition during the release period such as, for example, to increase the release rate in specific parts of the gastro-intestinal system of a patient.

Surprisingly the present inventors found that by an addition to a controlled release pharmaceutical composition comprising an active material embedded in a lipophilic matrix, of an insoluble, organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, the release rate of an active material can be selectively increased in a later stage of the release period, i.e. after about three hours of the beginning of the release period an increase in the release rate of the active material can be achieved.

### Summary of the Invention

In a first aspect, therefore, the present invention provides a process for a selective increase of a release rate in a later stage of the release period of an active material from a controlled release pharmaceutical composition comprising an active material and a lipid material embedding said active material, comprising the step of:
adding a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, in an amount of 3 to 20 percent by weight, based on the entire weight of the controlled release pharmaceutical composition, to a lipophilic matrix comprising an active material embedded in a lipid material.

In a second aspect the present invention relates to a controlled release pharmaceutical composition comprising an active material and a lipid material embedding said active material, characterized in that it further comprises a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, in an amount of 3 to 20 percent by weight, based on the entire weight of the controlled release pharmaceutical composition.

In a third aspect, therefore, the present invention provides a use a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, for selectively increasing a release rate in a later stage of the release period of an active material from a controlled release pharmaceutical composition comprising an active material and a lipid material embedding said active material.

### Description of the Drawings

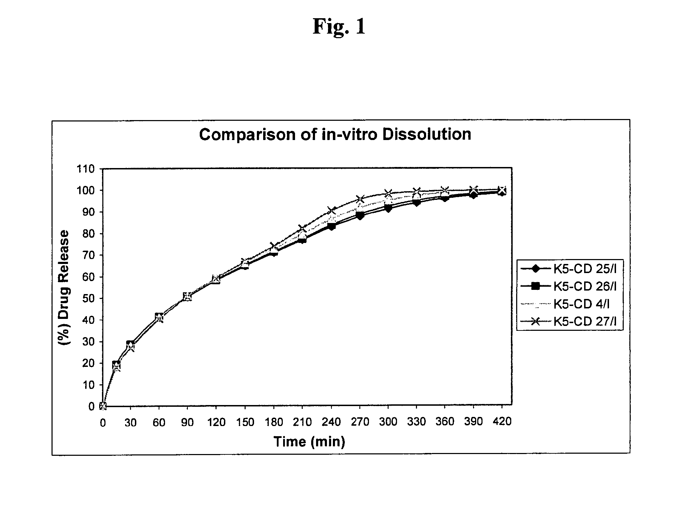

Fig. 1 shows a release profile of diltiazem hydrochloride in an aqueous medium (acetate buffer pH 4.5) from controlled release pharmaceutical compositions prepared in example 1.

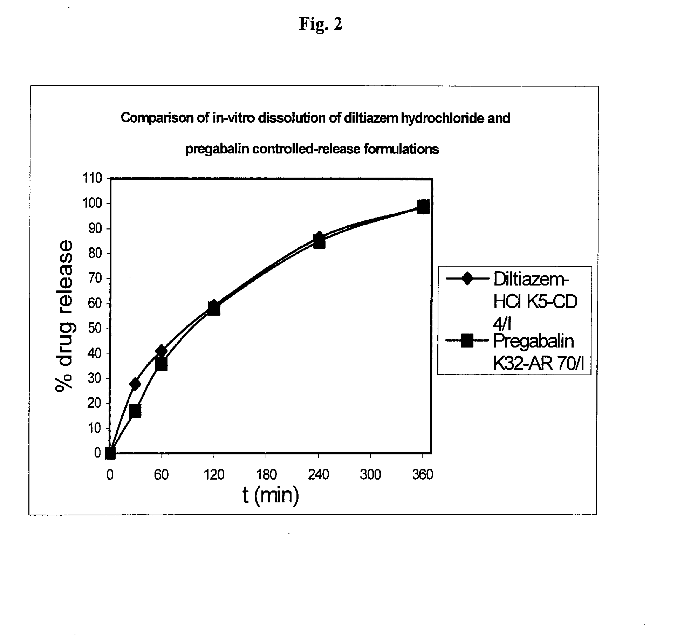

Fig. 2 shows a release profile of diltiazem hydrochloride and pregabalin in an aqueous medium from controlled release pharmaceutical compositions prepared in example 2.

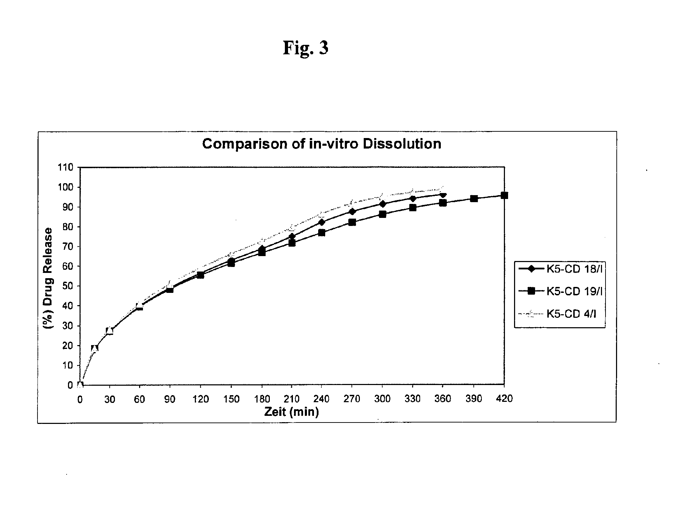

Fig. 3 shows a release profile of diltiazem hydrochloride in an aqueous medium (acetate buffer pH 4.5) from controlled release pharmaceutical compositions prepared in example 3.

### Description of the Preferred Embodiments

In one aspect the present invention provides a process for a selective increase of a release rate in a later stage of the release period of an active material from a controlled release pharmaceutical composition comprising an active material and a lipid material embedding said active material, comprising the step of:
adding a swellable organic disintegrant as defined above, in an amount of 3 to 20 percent by weight, based on the entire weight of the controlled release pharmaceutical composition, to a lipophilic matrix comprising an active material embedded in a lipid material.

The percent of the ingredient required in the pharmaceutical composition of the present invention, e.g. the active material, the lipid material, the disintegrant and other ingredients are calculated on a dry weight basis.

In a further aspect the present invention relates to a controlled release pharmaceutical composition comprising an active material and a lipid material embedding said active material, characterized in that it further comprises a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, in an amount of 3 to 20 percent by weight, based on the entire weight of the controlled release pharmaceutical composition.

For purposes of the present invention, the term "controlled release" means that the therapeutically active material or drug is released from the pharmaceutical composition at a controlled rate such that therapeutically beneficial blood levels (but below toxic levels) of the active material are maintained over an extended period of time, e.g., providing a 10, 12, 16 or 24 hour dosage form.

The present pharmaceutical composition comprises a pharmaceutical composition in unit dosage form. The term "unit dosage form", as employed herein, refers to a physically discrete unit suitable as unitary dosage to mammals, including humans, with each unit containing a predetermined quantity of active material calculated to produce the desired effect in association with the carrier, disintegrant and other ingredients of the formulation as described herein.

The present pharmaceutical composition is applicable to a wide variety of drugs or active materials suitable for use in controlled release pharmaceutical compositions selected from a group of active materials ranging from very soluble active materials (1 g of active material is soluble in less than 1 ml of solvent such as water) (e.g. Diltiazem® hydrochloride) to sparingly soluble active materials (1 g of active material is soluble in 30 to 100 ml solvent such as water) (e.g. pregabalin) as defined in the European Pharmacopoeia (European Pharmacopoeia, 4^{th} edition, 2002, Council of Europe, Strasbourg/France).

Representative active materials falling within the above mentioned definition may include antacids, anti-inflammatory substances, coronary vasodilators, cerebral vasodilators, psychotropics, antimanics, stimulants, anti-histamines, laxatives, decongestants, vitamins, gastrointestinal sedatives, anti-diarrheal preparations, antianginal drugs, vasodilator anti-arrythmics, anti-hypertensive drugs, vasoconstrictors, migraine treating agents, anticoagulants, anti-thrombotic drugs, analgetics, anti-pyretics, hypnotics, sedatives, anti-emetics, anti-nauseants, anticonvulsants, neuromuscular drugs, hyper- and hypoglycemic agents, thyroid and anti-thyroid preparations, diuretics, anti-spasmodics, uterine relaxants, mineral ans nutritional additives, anti-obesity drugs, anabolic drugs, erythropoietic drugs, anti-astmatics, expectorants, cough suppressants, mucolytics, anti-uricemic drugs, and other drugs or active materials acting locally in the mouth, such as topical analgetics, or a combination thereof and the like. The present pharmaceutical composition may contain more than one active material.

The active materials are present in pharmaceutically effective amounts. It is preferred that the active material be present in amounts ranging from about 0.5 percent by weight to about 80 percent by weight, preferably to about 65 percent by weight, based on the entire weight of the controlled release pharmaceutical composition.

As a lipid material to be used according to the present invention there may be used one or more lipid or lipoid materials normally employed which have a melting point in the range of from 40 to 100°C. Typically water-insoluble support materials for example fatty alcohols and especially higher alkanols containing more than 13 und especially 16 to 22 carbon atoms such as cetyl and stearyl alcohol, as well as mixtures thereof may be used. Use can also be made of fatty acids which bring about a liberation of the active material dependent on the pH, especially higher alkane-carboxylic acids, for example stearic acid.

Glycerides, especially hydrogenated cottonseed oil or castor oil, as well as mono-, di- or triesters of glycerol with palmitic acid or stearic acid or behenic acid or mixtures thereof can also be used. Furthermore, pulverized, wax-like materials of vegetable, animal, mineral or synthetic origin can be used. The lipophilic salts of fatty acids, such as magnesium stearate, are also very suitable. It is only necessary that the embedding lipid or lipoid material is stable in the intended temperature range, is physiologically inert, and does not react with the pharmaceutically active material.

The release rate of the active material surprisingly behaves as described above largely irrespective of the kind of active material used, that means that the release rate of the active material is not changed at the beginning, but only during a later stage of the release period by using the disintegrant to be used according to the present invention even if active materials to be released belong to different solubility classes.

The disintegrant to be used, according to the present invention, is selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein. Preferably, maize starch is used as the disintegrant according to the present invention.

As mentioned above, by adding one or more of the disintegrants mentioned above, the release rate of a controlled release pharmaceutical composition in a later stage of the release period can be selectively increased while the release rate at the beginning of the release period is maintained at the same level as for a controlled release composition which does not comprise such a disintegrant as claimed according to the present invention.

For the purposes of the present invention, the term "selective increase in the release rate in a later stage of the release period" means that the release rate of an active material from a controlled release pharmaceutical composition is maintained within the first two hours of the entire release period at about the same level as for a controlled release pharmaceutical composition, which does not comprise the disintegrant used according to the present invention, but is increased in a period of the third to seventh hour of the entire release period compared to a controlled release pharmaceutical composition which does not comprise the disintegrant used according to the present invention.

This means that in a period of the third to seventh hour, preferably the third to sixth hour, of the entire release period by using the disintegrant to be used according to the present invention, a rise in the release rate preferably of about 5%, more preferably of about 7%, most preferably of about 10%, compared to a controlled release pharmaceutical composition which does not comprise the disintegrant used according to the present invention, is achieved.

The present formulation also may contain optional components. For example, although not necessary, in a preferred embodiment, the present formulation additionally contains a lubricant that is typically used in the pharmaceutical arts for oral tablets. As used herein, the term "lubricant" refers to a material which can reduce the friction between the diewalls and the punch faces which occurs during the compression and ejection of a tablet. The lubricant prevents sticking of the tablet material to the punch faces and the die walls.

As used herein, the term "lubricant" includes anti-adherents. Examples of lubricants include stearate salts, e.g., alkaline earth, and transition metal salts thereof, e.g. calcium, magnesium, or zinc; stearic acid, polyethylene oxide, talc, hydrogenated vegetable oil, and vegetable oil derivatives, silica, silicones, high molecular weight polyalkylene glycol, e.g. high molecular weight polyethylene glycol, monoesters of propylene glycol, saturated fatty acid containing about 8-22 carbon atoms and preferably 16-20 carbon atoms. The preferred lubricants are the stearate salts, stearic acid, talc and the like. To avoid tablet sticking during formation and/or ejection, the present formulation contemplates utilizing a lubricating effective amount of the lubricant. Preferably, the lubricant is present in amounts ranging from about 0.1% to about 5% by weight, and more preferably from about 1% to about 4% by weight of the tablet.

Another optional ingredient is an inert filler. The filler may be substantially water soluble or water insoluble. A filler is used if needed or desired, although it is not necessary for the present formulation. The fillers used in the present formulation are those typically used in the pharmaceutical arts for oral tablets. Examples include calcium salts, such as calcium sulfate, dicalcium phosphate, tricalcium phosphate, calcium lactate, calcium gluconate, and the like, glycerol phosphate; citrates; and mixture thereof, and the like. However, the inert filler of the controlled release formulation of the present invention preferably comprises a pharmaceutically acceptable saccharide, including a monosaccharide, a disaccharide, or a polyhydric alcohol and/or mixture of any of the foregoing. Examples thereof include sucrose, dextrose, lactose, microcrystalline cellulose, fructose, xylitol, sorbitol, mixtures thereof and the like. The filler, if present, is present in amounts ranging from about 1% to about 90% by weight.

Other optional ingredients, that are also typically used in pharmaceuticals, may also be present, such as coloring agents, preservatives (e.g. methyl parabeans), artificial sweeteners, flavorants, antioxidizing agents and the like. Artificial sweeteners include, but are not limited to, saccharin sodium, aspartame, dipotassium glycyrrhizinate, stevia, thaumatin and the like. Flavorants include, but are not limited to, lemon, lime, orange and menthol. The colorants include, but are not limited to, various food colors, e.g. FD & C colors, such as FD & C Yellow No. 6, food lakes and the like. Examples of anti-oxidants include ascorbic acid, sodium metabisulphite, and the like. These optional ingredients, if present, are preferably present in amounts ranging from about 0.1% to about 5% by weight of the tablet and most preferably less than about 3% (w/w) of the tablet.

The pharmaceutical composition of the present invention is usually prepared by using a partial extrusion process. Said process comprises a first step of extruding an active material with a lipid material. Hereby, the above mentioned ingredients may be mixed in a typical blender that is normally utilized in the pharmaceutical arts, such as a Hobart mixer, V-blender, a planetary mixer, drum hoop mixers, shaker mixers, Twin shell blender and the like. Mixing can also be done directly in an extruder, depending on the extruder design (e.g. a twin-screw extruder with two separately controllable gravimetric feeders and mixing elements at the extruder screws) and the temperatures applied in the mixing zone of the extruder (below the melting point of meltable ingredients). Thereafter, the ingredients are extruded by using an extruder such as a twin-screw extruder typically at temperatures ranging from about 60°C to about 90°C. Thereafter, the homogeneous composition obtained can be cooled, and the solid extrudate may be granulated.

The granulated extrudate then is preferably intimately mixed with the disintegrant and other optional ingredients mentioned above in a large batch using techniques well known in the pharmaceutical arts until the mixture is homogenous.

The term "homogenous" is used to denote that the various components are substantially uniform throughout the invention, i.e. a substantially homogeneous blend is formed.

When the mixture is homogenous, a unit dosage amount of the mixture can be compressed into a tablet form using a tablet machine typically utilized in the pharmaceutical arts. More specifically, the mixture is fed to the die of a tablet press and sufficient pressure is applied to form a solid tablet. Such pressure can vary, and typically ranges from about 1,000 psi (6.9 MPa) to about 6,000 psi (41.4 MPa) and preferably about 2,000 psi (13.8 MPa) compression pressure. The solid pharmaceutical composition according to the present invention is compressed to a sufficient hardness to prevent the premature ingress of the aqueous medium into the tablet. Preferably, the pharmaceutical composition is compressed into a tablet form which is of the order of 5-25 Kp (50 - 250 N) and more, preferably 8-20 Kp (80 - 200 N) as determined by a Schleuniger hardness test.

The substantially uniformly blended mixture may optionally be milled, e.g. passed through a screen, sieve, etc. to reduce the size of the particles thereof. The screen or sieve, and the like, is preferably less than about 140 mesh, and more preferably less than about 100 mesh, and even more preferably, less than about 40 mesh, and most preferably less than about 20 mesh.

Next, the blend is dried. In this step, the solvent is removed from the blend by physical means known to the skilled artisan, e.g. by evaporation. The resulting granules are again milled, e.g. passed through a screen or sieve to further reduce the size of the particles to the desired size.

After the tablet is formed, the tablet can be coated with materials normally used in pharmaceuticals, if desired. If coated, the coating is prepared by techniques known in the art. However, the formulation of the present invention is preferably uncoated.

A tablet product is obtained which has the desired hardness and friability typically found for pharmaceutical tablets. The hardness is preferably 5-25 Kp (50 - 250 N) and more preferably 8-20 Kp (80 - 200 N). The present formulation in tablet form has an excellent drug release profile. More specifically, it has a predetermined controlled and sustained action and a regular delayed pattern so that the medicament is available over a period of up to 36 hours, depending upon the precise tablet size, the identity of the active material, aqueous solubility of the active material, hardness and the particular carrier composition. For example, in accordance with the process of the present invention, a controlled release diet supplement can be prepared wherein the release time is 8-10 hours, 15-18 hours, 20-24 hours, etc. as desired. Furthermore, the release profile of each pharmaceutical composition is substantially uniform. Finally, the tablets prepared in accordance with the present invention are hard and dense, have low friability and provide controlled and sustained release over an extended period. Solid dry forms prepared by the present invention are stable and their release rate does not change to any significant (if any) extent over an extended period of storage.

The controlled release pharmaceutical composition is provided in solid form, conveniently in a unit dosage form. Provision of the controlled release medicament in solid unit dosage form for oral administration, especially in tablet form, is preferred. Preferably, it is intended to release the pharmacologically active material in the above mentioned way after ingestion within the body as the formulation progresses along the gastro-intestinal tract. In this regard, the gastro-intestinal tract is considered to be the abdominal portion of the alimentary canal, i.e. the lower end of the esophagus, the stomach and the intestines.

The dosages of a formulation according to the invention correspond to the normal dosages of the particular active ingredient known to the skilled artisan. The precise amount of active material or drug administered to a patient will depend on a number of factors, including the age of the patient, the severity of the condition and the past medical history, among other factors, and always lie within the sound discretion of the administering physician. As a guideline for a suitable dosage, reference is made to the Physicians Desk Reference.

Unless indicated to the contrary, all percentages are weight percentages relative to the tablet.

The following non-limiting examples further illustrate the present invention.

### Example 1: Influence of maize starch on the release rate of pharmaceutical compositions

### Production process:

1.) In a first step 7.12 kg of a common extrudate composed of diltiazem hydrochloride (4 parts) and glycerol behenate (1 part) were prepared. In detail, the two starting materials were weighed and then mixed for 10 minutes in an Engelsmann Rhönradmischer ELTE 650 mixer. Thereafter, the mixture was extruded in a co-rotating twin-screw extruder Haake Rheomex PTW 16 / 25 p at a temperature of 80 to 85°C. Thereafter, the homogenous composition was cooled on a moving belt. After an additional cooling period the solidified extrudate was granulated through a 1.25 mm sieve of a Frewitt GLA granulating machine.
2.) Tablet charges (charge amount 580 g each) having different quantitative compositions (see Table 1 below) were prepared from the common extrudate. In detail, the optional ingredients mannitol, microcrystalline cellulose, maize starch, highly disperse silica were weighed, added through a 1 mm hand sieve to the weighed extrudate and mixed with a T2C Turbula mixer for 5 minutes. Thereafter, a weighed amount of magnesium stearate was added through a 0.5 mm hand sieve to the mixture and the entire composition was mixed in the same mixer for 5 minutes. The resulting mixture was compressed by using a Korsch EKO-DMS instrumented tablet press having a 16 x 9 mm die and a curvature of 11 mm.

**Table 1:**

| Formulations having a different content of maize starch | | | | | | | | |
|---|---|---|---|---|---|---|---|---|
| | mg | amount(%) | mg | amount(%) | mg | amount(%) | mg | amount(%) |
| Diltiazem-HCl | 300.0 | 51.73 | 300.0 | 51.73 | 300.0 | 51.73 | 300.0 | 51.73 |
| Glycerolbehenate | 75.0 | 12.93 | 75.0 | 12.93 | 75.0 | 12.93 | 75.0 | 12.93 |
| Mannitol | 84.0 | 14.48 | 74.0 | 12.76 | 64.0 | 11.03 | 54.0 | 9.31 |
| microcrystalline cellulose | 100.0 | 17.24 | 100.0 | 17.24 | 100.0 | 17.24 | 100.0 | 17.24 |
| **Maize starch** | **10.0** | **1.72** | **20.0** | **3.44** | **30.0** | **5.17** | **40.0** | **6.90** |
| Highly disperse SiO₂ | 5.0 | 0.86 | 5.0 | 0.86 | 5.0 | 0.86 | 5.0 | 0.86 |
| Mg-stearate | 6.0 | 1.04 | 6.0 | 1.04 | 6.0 | 1.04 | 6.0 | 1.04 |
| Total | 580.0 | 100.0 | 580.0 | 100.0 | 580.0 | 100.0 | 580.0 | 100.0 |
| Sample No. | K5-CD 25/I | | K5-CD 26/I | | K5-CD 4/I | | K5-CD 27/I | |

→ compensation through the amount of mannitol

The amount of maize starch is varied in a range of from 1.7% to 6.9%.

### Results of the release rate:

Surprisingly, it was found that the release rate of active material was controlled by maize starch in such a way that the selective increase of a release rate in a period between the 3^{rd} and the 7^{th} hour of the release period of an active material was achieved.

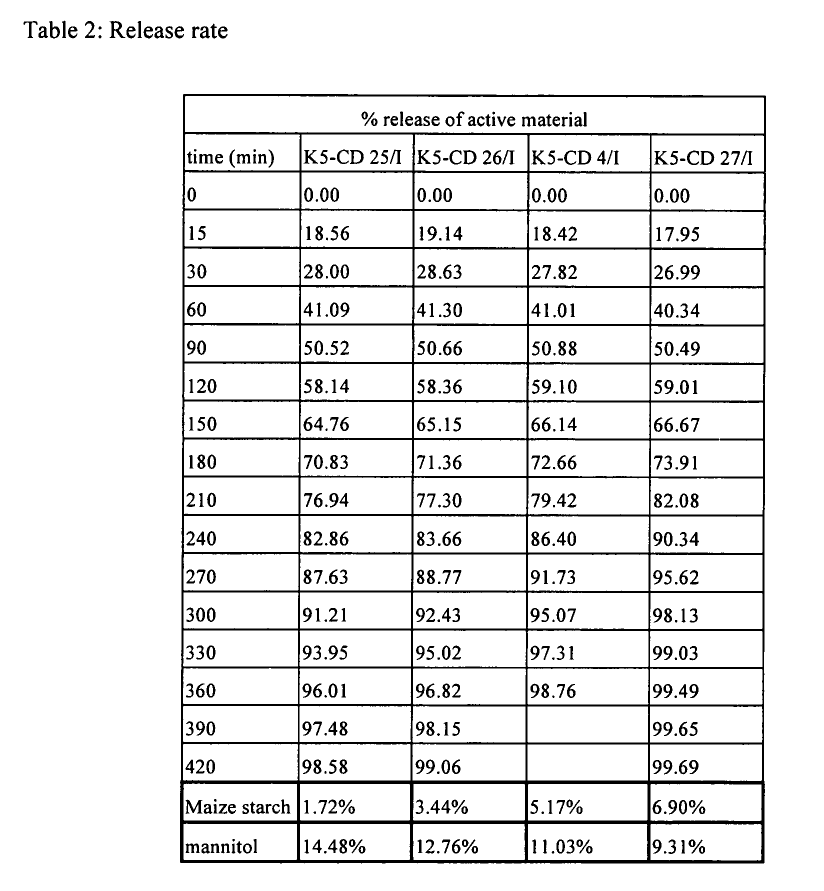

The release rate can be selectively controlled within the period mentioned above by using different amounts of the disintegrant maize starch. During the first two hours of the release period significant changes (changes 1 % at a maximum) in the release rate could not be observed. Thereafter, however, significant differences could be seen, which amount up to 9% after 4.5 hours of the release period. Thus, by using a disintegrant claimed according to the present invention, the form of the release curve can be clearly modified.

### Example 2

This example demonstrates that the effect shown in example 1 is achieved with a large amount of different active materials selected from the group ranging from very water-soluble active materials (1 g of active material is soluble in less than 1 ml of water) to sparingly water-soluble active materials (1 g of active material is soluble in 30 to 100 ml water) (see also the conditions with respect to water solubility given in the European Pharmacopoeia mentioned above).

In the following, pregabalin and diltiazem hydrochloride were used:
a) Pregabalin
   Struktural formula of pregabalin 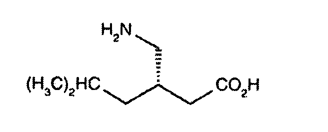 solubility 32,1 mg/ml in water, i.e. 1 g of pregabalin is soluble in 31 ml water, thus said active material is considered a sparingly soluble material according to the definition of the European Pharmacopoeia.
b) diltiazem hydrochloride
   Struktural formula of diltiazem hydrochloride 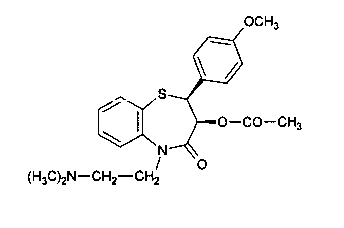 This active material is freely soluble in water according to the European Pharmacopoeia.

### Production process:

1.) In a first step an extrudate composed of diltiazem hydrochloride or pregabalin (4 parts) and glycerol behenate (1 part) was prepared. In detail, the two starting materials were weighed and they were mixed for 10 minutes in an Engelsmann Rhönradmischer ELTE 650 mixer . Thereafter, the mixture was extruded in a Haake Rheomex PTW 16 / 25 p co-rotating twin-screw extruder at a temperature of 80 to 85°C. Thereafter, the homogenous composition was cooled on a moving belt. After an additional cooling period the solidified extrudate was granulated through a 1.25 mm sieve of a Frewitt GLA granulating machine.
2.) Tablet charges (charge amount 580 g each) having different quantitative compositions (see table 3 below) were prepared from the extrudate. In detail, the optional ingredients mannitol, microcrystalline cellulose, maize starch, and highly disperse silica were weighed, added through a 1 mm hand sieve to the weighed extrudate and mixed with a T2C Turbula mixer for 5 minutes. Thereafter, a weighed amount of magnesium stearate was added through a 0.5 mm hand sieve to the mixture and the entire composition was mixed in the same mixer for 5 minutes. The resulting mixture was compressed by using an instrumented Korsch EKO-DMS tablet press having a 16 x 9 mm die and a curvature of 11 mm.

**Table 3:**

| composition of the pharmaceutical compositions | | | | |
|---|---|---|---|---|
| | pregabaline (mg) | Content (%) | diltiazem HCl (mg) | Content (%) |
| Active material | 300.0 | 51.73 | 300.0 | 51.73 |
| Glycerol behenate | 75.0 | 12.93 | 75.0 | 12.93 |
| Mannitol | 64.0 | 11.03 | 64.0 | 11.03 |
| Microcrystalline cellulose | 100.0 | 17.24 | 100.0 | 17.24 |
| Maize starch | 30.0 | 5.17 | 30.0 | 5.17 |
| Highly disperse SiO₂ | 5.0 | 0.86 | 5.0 | 0.86 |
| Mg-Stearate | 6.0 | 1.04 | 6.0 | 1.04 |
| Total | 580.0 | 100.0 | 580.0 | 100.0 |
| Sample No. | K72-AR70/I | | K5-CD 4/1 | |

### Results of the release rate:

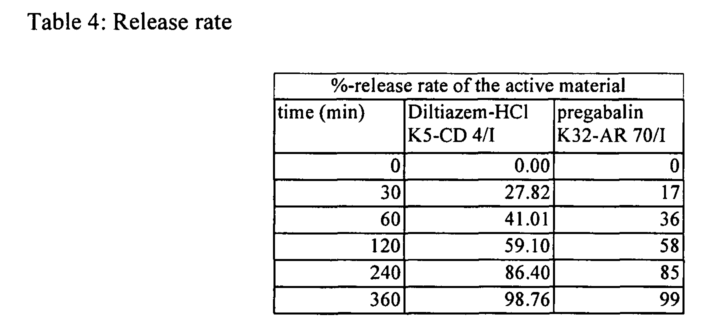

### Example 3: Variation of the amount of an easily soluble hydrophilic disintegrant of the state of the art

**Table 5:**

| Pharmaceutical formulations | | | | | | |
|---|---|---|---|---|---|---|
| | mg | amount (%) | mg | amount (%) | mg | amount (%) |
| Extrudate | | | | | | |
| **Diltiazem-HCl** | **300.0** | **51.73** | **300.0** | **55.79** | **300.0** | **60.55** |
| Glycerol behenate | 75.0 | 12.93 | 75.0 | 13.95 | 75.0 | 15.14 |

| Tabletting mixture | | | | | | |
|---|---|---|---|---|---|---|
| **Mannitol** | **64.0** | **11.03** | **32.0** | **5.95** | **---** | **---** |
| microcrystalline cellulose | 100.0 | 17.24 | 92.7 | 17.24 | 85.4 | 17.24 |
| Maize starch | 30.0 | 5.17 | 27.8 | 5.17 | 25.6 | 5.17 |
| Highly disperse SiO₂ | 5.0 | 0.86 | 4.6 | 0.86 | 4.3 | 0.86 |
| Mg-Stearate | 6.0 | 1.04 | 5.6 | 1.04 | 5.1 | 1.04 |
| Total | 580.0 | 100.0 | 537.7 | 100.0 | 495.4 | 100.0 |
| Sample No. | K5-CD 4/I | | K5-CD 18/I | | K5-CD 19/I | |

→ the other ingredients were used in the same percentages

By reducing the amount of a very soluble hydrophilic adjuvant mannitol (from 11 to 0%) the ratio of the active material can be increased from 51% to 60%.

### Production process:

1.) In a first step an extrudate composed of diltiazem hydrochloride (4 parts) and glycerol behenate (1 part) was prepared. In detail, the two starting materials were weighed and they were mixed for 10 minutes in an Engelsmann Rhönradmischer ELTE 650 mixer. Thereafter, the mixture was extruded in a Haake Rheomex PTW 16 / 25 p co-rotating twin-screw extruder at a temperature of 80 to 85°C. Thereafter, the homogenous composition was cooled on a moving belt. After an additional cooling period the solidified extrudate was granulated through a 1.25 mm sieve of a granulating machine Frewitt GLA.
2.) Tablet charges (charge amount 580 g each) having different quantitative compositions (see table 5 above) were prepared from the extrudate. In detail, the optional ingredients mannitol (if necessary), microcrystalline cellulose, maize starch, and highly disperse silica were weighed, added through a 1 mm hand sieve to the weighed extrudate and mixed with a T2C Turbula mixer for 5 minutes. Thereafter, a weighed amount of magnesium stearate was added through a 0.5 mm hand sieve to the mixture and the entire composition was mixed in the same mixer for 5 minutes. The resulting mixture was compressed by using an instrumented Korsch EKO-DMS tablet press having a 16 x 9 mm die and a curvature of 11 mm.

### Results of the release rate:

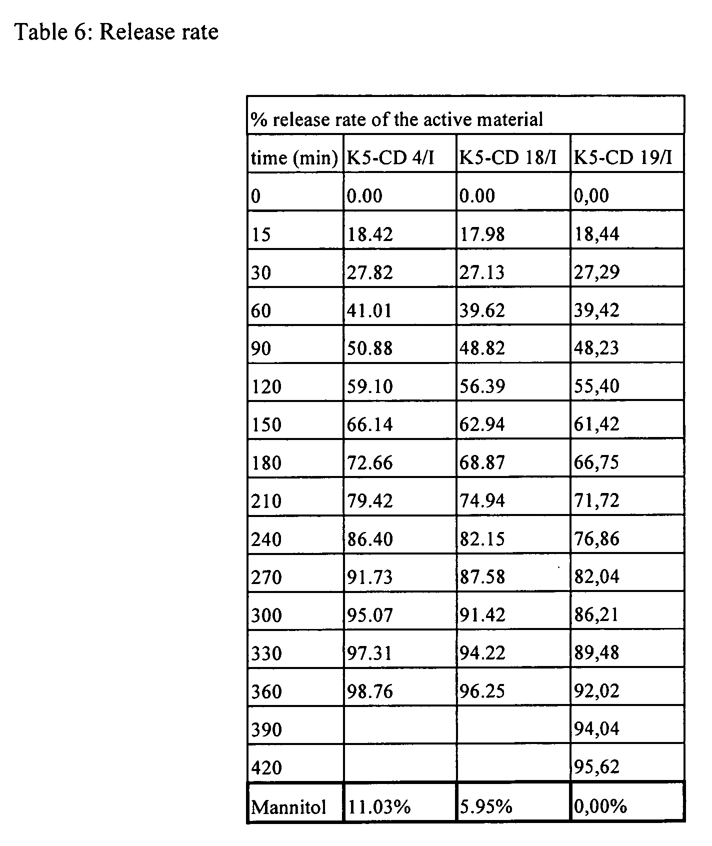

Contrary to the use of a disintegrant to be used according to the present invention, addition of differing amounts of a soluble hydrophilic ingredient such as mannitol, give rise to a significant change (4% in the maximum) in the release rate of the active material already in the first two hours of the release period. Said change will have a continuous influence on the release rate of the active material (an increase of up to 10% after 4.5 h).

In contrast, a disintegrant to be used, according to the present invention, will only selectively modify the release rate in a later stage of the release period of the active material.

## Claims

1. A process for a selective increase of a release rate in a later stage of the release period of an active material from a controlled release pharmaceutical composition comprising an active material and a lipid material embedding said active material, comprising the step of:
adding a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, in an amount of 3 to 20 percent by weight, based on the entire weight of the controlled release pharmaceutical composition, to a lipophilic matrix comprising an active material embedded in a lipid material.

2. A controlled release pharmaceutical composition comprising an active material and a lipid material embedding said active material, **characterized in that** it further comprises a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, in an amount of 3 to 20 percent by weight, based on the entire weight of the controlled release pharmaceutical composition.

3. A use a swellable organic disintegrant selected from the group consisting of potato starch, maize starch, wheat starch, tapioca starch, pregelatinized starch, physically modified amylose, modified potato starch, amylopectin, sodium starch glycolate, sodium amylopectin glycolate, croscarmellose sodium, carmellose calcium, calcium alginate, crosslinked dextran and formaldehyde-casein, for selectively increasing a release rate in a later stage of the release period of an active material from a controlled release pharmaceutical composition comprising an active material and a lipid material embedding said active material.
